# EUROPÄISCHE PATENTANMELDUNG

(11) **EP 0 627 222 A1**
(43) Veröffentlichungstag der Anmeldung: **07.12.1994**
(21) Anmeldenummer: 94107898.2
(22) Anmeldetag: 21.05.1994
(51) Int. Cl.: A61K 31/57, A61K 47/26

(54) **Infusions- und Injektionspräparate enthaltend Dexamethason-21-Phosphat-di-Natrium Salz und Polysorbate**

(30) Priorität: 03.06.1993 DE 4318398
(71) Anmelder: MERCK PATENT GmbH, D-64293 Darmstadt (DE)
(72) Erfinder: Hakert, Hubertus, Dr., D-64846-Gross-Zimmern (DE); Krüger, Ludwig, D-63741 Aschaffenburg (DE); Schäffler, Achim, Dr., D-64743 Beerfelden (DE)

(57) **Zusammenfassung**

Infusions- und Injektionspräparate in hochkonzentrierter Form, enthaltend Dexamethason-21-phosphat-di-natriumsalz sowie Polysorbate.

## Beschreibung

Die Erfindung betrifft Präparate zur Injektion bzw. zur Infusionstherapie, die Dexamethason-21-phosphat-di-natriumsalz sowie Polysorbate enthalten.

Dexamethasonphosphat-Salze werden in der Medizin schon seit längerer Zeit zur parenteralen Corticoid-Therapie eingesetzt. Derartige Injektions- oder Infusionspräparate sind aus herstellungsbedingten Gründen ausschließlich in Formen erhältlich, in denen der Wirkstoff in niedrigen Konzentrationen vorliegt. Man kennt beispielsweise Zubereitungen mit 4 bis 10 mg Wirkstoff je ml Lösungsmittel. In vielen Fällen ist es jedoch wünschenswert und notwendig, das Corticoid bzw. eines seiner Salze in einer höheren Dosierung zu injizieren.

Die Anwendbarkeit der Dexamethasonphosphat-Salzlösungen in der Medizin ist aufgrund der geringen Löslichkeit der Salze beträchtlich eingeschränkt. Es konnte bisher nicht erreicht werden, ein geeignetes Verfahren zur Herstellung hochkonzentrierter Zubereitungen aufzufinden.

Bisherige Injektionspräparate, die den Wirkstoff in hoher Konzentration enthalten, konnten nicht verwendet werden, da es regelmäßig zu partikulären Ausscheidungen des Dexamethasonphosphats bzw. seiner Salze kam.

Die Anforderungen, die an injizierbare Präparate gestellt werden, konnten durch bisherige hochkonzentrierte Dexamethasonphosphat-Salzlösungen nicht erfüllt werden, da keine homogenen Lösungen herstellbar waren.

Der Erfindung lag die Aufgabe zugrunde, Dexamethasonphosphat-di-natriumsalz-haltige Lösungen in hochkonzentrierter Form bereitzustellen, die zur parenteralen Anwendung geeignet sind, da sie die zuvor genannten Nachteile der Ausscheidung von partikulären Verunreinigungen nicht aufweisen, somit homogen sind und auch bei Lagerung eine Stabilität gewährleisten, ohne daß sich der Wirkstoff in fester Form abscheidet.

Es wurde gefunden, daß der Zusatz von geringen Mengen Polysorbaten die Löslichkeit des Wirkstoffes erheblich erhöht und die Problematik der partikulären Ausscheidung in hochkonzentrierten Präparaten zur Infusion bzw. Injektion vollständig beseitigt. Es ist somit möglich, hochkonzentrierte Zubereitungen mit niedrigen Volumina bereitzustellen, die einen flexiblen Einsatz, eine Lagerung in Notfallkoffern und eine schnelle hohe Dosierung bzw. eine individuelle Dosierung, je nach Anforderung durch Verdünnung, z.B. mit anderen Infusionslösungen, ermöglichen.

Gegenstand der Erfindung sind Injektions- und Infusionspräparate, enthaltend Dexamethason-21-phosphat-di-natriumsalz, dadurch gekennzeichnet, daß es Polysorbate enthält.

Als Polysorbate eignen sich vorzugsweise ethoxylierte Sorbitanester, wie Oleate, Stearate, Laurate oder Palmitate, insbesondere Sorbitanmonooleat (Polyoxyethylen-80 Tween 80®). Es ist jedoch ebenso möglich, Mischungen der einzelnen Polysorbate einzusetzen.

Gewöhnlich werden 0,5-2 Gew.-% Polysorbat, vorzugsweise 0,8-1,5 Gew.-%, besonders bevorzugt 0,9-1,3 Gew.-%, bezogen auf die eingesetzte Wirkstoffmenge, verwendet.

Als Lösungsmittel dient in der Regel Wasser. Es ist aber auch möglich, den Wirkstoff zunächst in geeigneten anderen Lösungsmitteln oder -gemischen , wie z.B. Alkoholen, insbesondere Ethanol, Propylenglykol, Polyethylenglykol, Benzylalkohol oder in Aminen, wie Triethanolamin, Diethanolamin, Ethylendiamin oder in Amiden, wie β-Hydroxyethyllactamid oder Diethylacetamid, zu lösen und diese Lösungen dann, nach Zugabe des Polysorbatanteiles, mit einer entsprechenden Menge Wasser zu verdünnen.

Im allgemeinen ist es angebracht, den löslich zu machenden Wirkstoff in einer geringen Menge Ethanol zu einer 0,2-10%igen Lösung zu lösen, dann mit 0,5-2 Gew.-% Polysorbat (bzgl. d. eingesetzten Wirkstoffmenge) zu versetzen und anschließend durch Zugabe von Wasser, gegebenenfalls von heißem Wasser von 40-90 °C, in eine wäßrige Lösung zu überführen.

Besonders bevorzugt ist jedoch die direkte Herstellung einer reinen wässerigen Lösung von Dexamethasonphosphat-di-natriumsalz. Hierzu wird Wasser, gegebenenfalls temperiertes Wasser von 40-90 °C, unter Rühren zu einer Mischung des Wirkstoffes und des Polysorbats hinzugegeben. Man verfährt in der Regel derart, daß man den Wirkstoff- und Polysorbat zunächst in 50-80 % der vorgesehenen Wassermenge löst und diese anschließend mit dem verbleibenden Teil des Lösungsmittels bis zum Endvolumen auffüllt. Anstelle eines Rührers kann ebenfalls ein Homogenisator verwendet werden.

Ebenso kann es in besonderen Fällen von Vorteil sein, die wäßrigen Lösungen durch Zusatz von Säuren oder Basen auf pH-Werte zwischen 4 und 8,5 einzustellen.

Auf diese Weise ist es möglich, Injektions- oder Infusionspräparate mit einer Konzentration von bis zu 500 mg Dexamethasonphosphat-di-natriumsalz pro ml Lösungsmittel herzustellen.

Die nach den zuvor geschilderten Methoden erhaltenen konzentrierten Lösungen können entsprechend ihrem Verwendungszweck unverdünnt oder gemischt mit isotonischen Mitteln, wie z.B. Natriumchloridlösungen oder Glucoselösungen, in verdünnter Form eingesetzt werden.

Die erfindungsgemäßen hochkonzentrierten Lösungen können injiziert oder infundiert werden. Sie sind mit herkömmlichen Infusionslösungen mischbar und können somit als Konzentrate diesen zugesetzt und anschließend als Mischung infundiert werden.

Die Injektions- oder Infusionspräparate gemäß der vorliegenden Erfindung können zusätzlich weitere Hilfs- oder Zusatzstoffe enthalten. Geeignete Additive sind beispielsweise Konservierungsmittel wie Parabene® oder Benzylalkohol, Puffersubstanzen zur Einstellung eines bestimmten pH-Wertes, Stabilisatoren wie EDTA oder Antioxidantien, Süß- oder Aromastoffe und/oder isotonische Zusätze, wie z.B. NaCl.

Die erfindungsgemäßen Injektions- und Infusionspräparate können selbstverständlich auch durch Zusatz von Wasser auf jede beliebige Zwischenkonzentration verdünnt werden.

Die dexamethasonphosphat-dinatrium-haltigen Präparate dienen zur Behandlung von Erkrankungen und/oder Mangelzuständen des menschlichen oder tierischen Körpers. Sie wirken hierbei insbesondere als Antiphlogistika, Antiarthritika und Antiallergika.

Sie können in verschiedenen Darreichungsformen wie z.B. Ampullen, Flaschen, insbesondere Infusionsflaschen, Injektionsgläsern oder Infusionsbeuteln bereitgestellt werden.

Neben den genannten Applikationsformen Injektionsglas, Infusionsflaschen und Ampulle ist es auch möglich, die Lösung in Fertigspritzen, Autoinjektionssysteme oder Pumpsysteme zu füllen und verschiedenen Behandlungen wie Sterilisation bei 121° oder Lyophilisation zu unterziehen.

Ebenso ist es möglich, die Lösungen entsprechend den Beispielen 2-16 in Inhalatoren an sich bekannter Bauart zu füllen, um bei der Anwendung eine pulmonale oder zentrale Wirkung des Kortikoides zu erreichen.

Die nachfolgenden Beispiele sollen die Erfindung näher erläutern, ohne diese einzuschränken. Bei allen vor- und nachstehenden Prozentangaben handelt es sich um Gewichtsprozente, während alle Temperaturen in °C angegeben sind. Bei dem verwendeten Wasser handelt es sich, soweit nicht anders angegeben, um injektionsfähiges Wasser.

### Beispiele

### Beispiel 1

In 800 ml Wasser werden 250 g Dexamethason-21-phosphat-di-natriumsalz und 5 g Tween 80® unter Rühren gelöst, bis eine klare Lösung entstanden ist. Anschließend verdünnt man mit weiteren 200 ml Wasser, rührt erneut 15 Minuten und filtriert durch einen Membranfilter. Man erhält eine wäßrige Dexamethason-21-phosphat-di-natriumsalzlösung mit einer Konzentration von 250 mg Wirkstoff pro ml Lösungsmittel.

### Beispiele 2-15

Analog Beispiel 1 erhält man die folgenden Lösungen (Volumen 1000 ml wäßrige Lösung):

| Bsp. | Dexa [g] | Tween 80® [mg] | C _{Dexa} [mg/ml] |
|---|---|---|---|
| 2 | 4 | 40 | 4 |
| 3 | 8 | 80 | 8 |
| 4 | 20 | 200 | 20 |
| 5 | 40 | 400 | 40 |
| 6 | 100 | 1000 | 100 |
| 7 | 150 | 1500 | 150 |
| 8 | 200 | 2000 | 200 |
| 9 | 250 | 2500 | 250 |
| 10 | 300 | 3000 | 300 |
| 11 | 350 | 4200 | 350 |
| 12 | 400 | 6400 | 400 |
| 13 | 100 | 1500 | 100 |
| 14 | 200 | 3000 | 200 |
| 15 | 300 | 5400 | 300 |
| Dexa: Dexamethason-21-phosphat-di-natriumsalz. | | | |

### Beispiel 16

Zu einem Gemisch von 0,5 g Tween 80® und 10 ml Ethanol werden 25 g Dexamethason-21-phosphat-di-natriumsalz gegeben. Nachdem man 1 Stunde gerührt hat, fügt man portionsweise auf 50° erhitztes Wasser bis zu einem Gesamtvolumen von 50 ml hinzu und setzt das Rühren fort, bis eine klare Lösung vorliegt. Anschließend filtriert man. Man erhält eine Lösung mit einer Wirkstoffkonzentration von 500 mg/ml.

Die nachstehenden Beispiele betreffen spezielle pharmazeutische Zubereitungen.

### Beispiel A: Injektionsgläser

Eine Lösung gemäß Beispiel 1 wird mit 2 N Salzsäure auf pH 6,5 eingestellt, steril filtriert, in Portionen von je 2 ml in Injektionsgläser abgefüllt, unter sterilen Bedingungen lyophilisiert und steril verschlossen. Jedes Injektionsglas enthält 500 mg Wirkstoff.

### Beispiel B: Infusionslösungen

Eine Lösung gemäß Beispiel 1 (Volumen 50 l) wird auf pH 6,8 eingestellt. Anschließend werden Portionen zu je 500 ml unter aseptischen Bedingungen in Infusionsflaschen gefüllt, lyophilisiert und unter sterilen Bedingungen verschlossen. Jede Infusionsflasche enthält 125 g Wirkstoff.

### Beispiel C: Ampullen

Eine Lösung gemäß Beispiel 1 wird unter aseptischen Bedingungen filtriert. Anschließend füllt man die Lösung zu Portionen von je 5 ml in Ampullen ab, verschließt unter sterilen Bedingungen und sterilisiert bei 121°. Jede Ampulle enthält 1,25 g Wirkstoff.

Analog können alle genannten pharmazeutischen Zubereitungen mit den Lösungen der Beispiele 2-16 erhalten werden. Darüber hinaus können auch hier nicht erwähnte Konzentrationen des Wirkstoffes durch Verdünnen mit Wasser erhalten werden.

## Patentansprüche

1. Infusions- und Injektionspräparate, enthaltend Dexamethason-21-phosphat-di-natriumsalz, dadurch gekennzeichnet, daß sie Polysorbate enthalten.

2. Infusions- und Injektionspräparate gemäß Anspruch 1, dadurch gekennzeichnet, daß die Konzentration des Wirkstoffes 1-500 mg pro ml Lösungsmittel beträgt.

3. Injektionspräparate gemaß der Ansprüche 1 und 2, dadurch gekennzeichnet, daß sie in unverdünnter Form injizierbar sind.

4. Infusions- und Injektionspräparate gemäß der Ansprüche 1 und 2, dadurch gekennzeichnet, daß sie mit handelsüblichen Infusionspräparaten mischbar sind.
